(19) Europäisches Patentamt
European Patent Office
Office européen des brevets

(11) Publication number: 0 309 271
A1

(12) # EUROPEAN PATENT APPLICATION

(21) Application number: 88308855.1

(22) Date of filing: 23.09.88

(51) Int. Cl.⁴: C 07 D 409/12
A 01 N 43/18
// C07D335/02

(30) Priority: 25.09.87 US 101321 25.09.87 US 101324

(43) Date of publication of application:
29.03.89 Bulletin 89/13

(84) Designated Contracting States:
CH DE FR GB GR LI

(71) Applicant: CHEVRON RESEARCH COMPANY
555 Market Street
San Francisco California 94105 (US)

(72) Inventor: Loh, William
804, Sartori Drive
Petaluma California 94952 (US)

Bassi, Pawan K.
432, Greenbrier Court
Benicia California 94510 (US)

(74) Representative: Kosmin, Gerald Emmanuel et al
HASELTINE, LAKE & CO. Hazlitt House 28 Southampton
Buildings Chancery Lane
London, WC2A 1AT (GB)

(54) **Selective herbicides able additionally to control lateral buds in tobacco plants.**

(57) 2-[1-[(5-chlorothienyl-2-yl)methoxyimino]ethyl]-3-hydroxy-5-(tetrahydro-2H-thiopyran-3-yl)-cyclohex-2-en-1-one and 3-alkanoyloxy derivatives and salts are disclosed. The compounds are useful to control sucker (lateral buds) in tobacco plants and also as selective herbicides against grasses.

EP 0 309 271 A1

**Description**

## SELECTIVE HERBICIDES ABLE ADDITIONALLY TO CONTROL LATERAL BUDS IN TOBACCO PLANTS

This invention relates to certain 2-[1-halothienylmethoxyimino)ethyl]-3-hydroxy-5-(tetrahydro-2H-thiopyranyl)-cyclohex-2-en-1-ones and salts and 3-alkanoyl derivatives thereof. This invention also relates to the use of such compounds to control tobacco suckers. In a further aspect, the invention relates to the use of such compounds as herbicides against grasses.

Tobacco crop management requires terminal growing meristem be removed so that the number of usable leaves and their quality can be optimized. The removal of the terminal meristem, a process called topping, encourages the rapid development of lateral buds. These lateral buds are known as suckers These suckers, if allowed to grow, can interfere with the proper development of usable tobacco leaves resulting in serious economic losses for the grower.

Manual removal of these suckers is very labor intensive. Chemicals are used, therefore, to arrest the development of lateral buds after topping. These chemicals mimic the phenomenon of apical dominance in tobacco cultivation after the plants are topped and allow the optimal development of usable leaves. For details on this subject, please refer to pages 233-262 of Principles of Flue-cured Tobacco Production by S. N. Hawks, Jr. and W. K. Collins, North Carolina State University (1983), and pages 71-81 of Plant Growth Regulating Chemicals, Vol. I, Ed. L. G. Nickel, CRC Press (1983).

In the chemical control of tobacco suckers, it is important that the chemical operates systemically; i.e., it should inhibit sucker development even when applied to other parts of the tobacco plant. This is important because in field-grown tobacco, the axillary buds, or suckers, are hidden in large leaf whorls. Thus, it is very difficult to apply the chemical spray such that it will directly contact the axillary buds. However, if the chemical operates systemically, this problem can be avoided because the chemical can be effectively applied to the leaves of the tobacco plant or even as a soil drench.

The most commonly used chemicals for tobacco sucker control are maleic hydrazide and flumetrain. Maleic hydrazide is a systemic inhibitor and though effective, it is not without its problems. Treatment with maleic hydrazide tends to leave undesirably high levels of residues in the leaves.

U.S. Patent No. 4,440,566, issued April 3, 1984, discloses compounds having the formula:

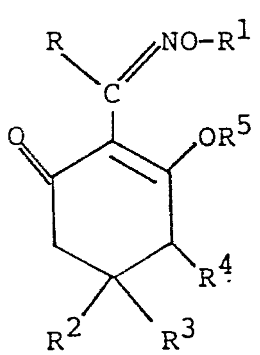

wherein R is most preferably alkyl of 1 to 3 carbon atoms, most preferably ethyl or propyl;
$R^1$ is most preferably 3-<u>trans</u>-chloroallyl or 4-chlorobenzyl;
$R^2$ and $R^3$ are preferably each alkyl of 1 to 3 carbon atoms or one of $R^2$ or $R^3$ is hydrogen and the other is alkylthioalkyl having 2 through 8 carbon atoms, most preferably $R^2$ and $R^3$ are each methyl or one of $R^2$ or $R^3$ is hydrogen and the other is 2-ethylthiopropyl.

This patent teaches that these compounds exhibit herbicidal activity against grasses and are safe with respect to broadleaf crops and also may be employed to prevent or retard the growth of lateral buds in plants and to promote the thinning out of fruit in various fruit trees.

U.S. Patent No. 4,624,696, issued November 25, 1986, discloses compounds having the formula:

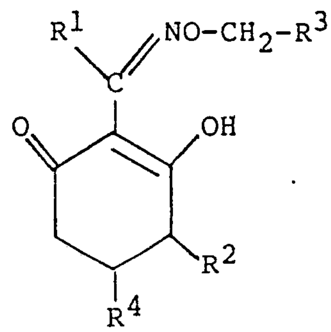

wherein $R^1$ is alkyl of 1 to 4 carbon atoms, $R^2$ is hydrogen or alkoxycarbonyl of 2 to 5 carbon atoms, $R^3$ is a 5-membered heterocyclic structure which contains 1 to 3 heteroatoms from the group consisting of N, O, and S and may contain 1 or 2 double bonds and 1 or 2 substituents from the group consisting of $C_1$-$C_4$-alkyl, $C_1$-$C_4$-alkoxy, halogen, trifluoromethyl, $C_1$-$C_4$ -alkoxymethyl, $C_1$-$C_4$-alkylthiomethyl, vinyl and phenyl, and $R^4$ is a 5-membered to 7-membered heterocyclic structure which contains one heteroatom or ring member, or two identical or different heteroatoms or ring members, from the group consisting of N, O, S, SO, and $SO_2$, may contain 1, 2, or 3 double bonds and is unsubstituted or substituted by not more than 2 alkyl or alkoxy groups, each of 1 to 4 carbon atoms, or is a radical of the Formula II

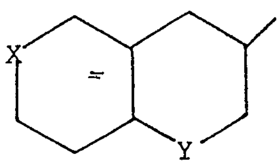

(II)

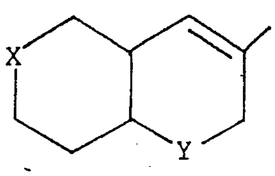

(IIa)

where X and Y are each N, O, S, SO, or $SO_2$, and salts of these compounds.

This patent teaches that $R^1$ is preferably a radical having two or three carbon atoms. The patent describes the compounds as exhibiting herbicidal activity against grasses and safe with respect to a number of enumerated crops.

The patent also teaches that these compounds exhibit herbicidal activity against grasses and are safe with respect to a number of crops.

U.S. Patent No. 4,596,877 discloses compounds having the formula

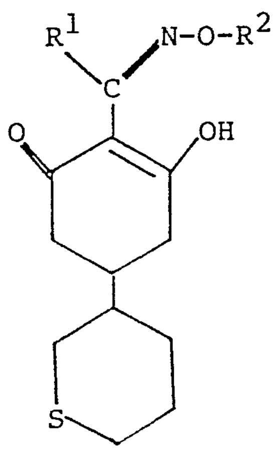

wherein $R^1$ is $C_1$-$C_4$ alkyl and $R^2$ is $C_3$-$C_5$ chloroalkenyl. $R^1$ is preferably ethyl or propyl.

The compounds are described as being effective to remove grasses while being safe for a number of crops.

U.S. Patent No. 4,432,786 discloses compounds having the following formula and their salts:

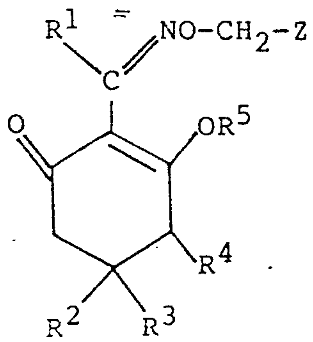

wherein $R^1$ is hydrogen or lower alkyl;
$R^2$ and $R^3$ are independently selected from the group consisting of hydrogen, lower alkyl, aryl having 6 through 10 carbon atoms (preferably phenyl), substituted aryl having 6 through 10 carbon atoms (preferably phenyl) and 1 through 4 substituents (preferably 1 or 2) independently selected from the group consisting of fluoro, chloro, bromo, iodo, or trifluoromethyl;
$R^4$ is hydrogen or alkoxycarbonyl having 2 through 4 carbon atoms;
$R^5$ is hydrogen, or an acyl group having 1 through 12 carbon atoms; and
Z is a group having the formula:

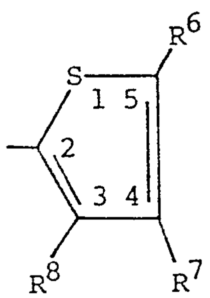

or

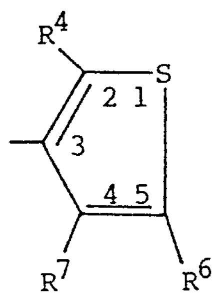

wherein $R^6$, $R^7$, and $R^8$ are independently selected from the group of hydrogen, halo, nitro, alkyl having 1 through 4 carbon atoms, alkoxy having 1 through 4 carbon atoms, or trifluoromethyl.

The compounds wherein $R^1$ is ethyl or propyl and/or Z is a chlorothienyl are preferred. The compounds are

described as exhibiting excellent grass herbicidal activity.

Certain 2-acyl-3-hydroxy-5-heterocyclecyclohexenone derivatives are described in German application DE 3,522,213 laid open January 2, 1987, as exhibiting a variety of plant growth regulating activities including preventing suckering in tobacco.

Commonly assigned U.S. application Serial No. 854,448 filed April 21, 1986, discloses that certain 2-[1-(3-chloroallyloxyimino)ethyl]-3-hydroxy-5-(2-substituted thioalkylidene)-cyclohex-2-en-1-one derivatives are useful to control tobacco suckers.

It has now been discovered that one of the 3-hydroxy-cyclohex-2-en-1-ones encompassed within the broad generic disclosure of U.S. Patent No. 4,624,696 (i.e., see Formula (1) below wherein R is hydrogen) and 3-alkanoyl esters thereof exhibit a surprising superior ability to control (inhibit) tobacco sucker growth. This activity is surprising and does not appear to exist throughout the class of 3-hydroxy and esterified 3-alkanoyl derivatives of 2-[1-(substituted-oxyimino)alkyl]-3-hydroxy-5-substituted-cyclohex-2-en-1-ones as a whole. At moderate dosage rate, the 3-alkanoyl ester derivatives also inhibit the growth of secondary sucker growth as well as primary sucker growth. These compounds are further readily absorbed and translocated within the tobacco plant so that the compounds do not have to directly contact the axillary buds to be effective. As discussed above, this affords a substantial advantage in applying the material.

It has also been discovered that the compound of Formula I below wherein R is hydrogen exhibits superior herbicidal activity as compared with others within the generic disclosure of U.S. Patent No. 4,624,696. The outstanding herbicidal activity possessed by this compound is particularly surprising because the substituent corre- ' sponding to the $R^1$ position in the compounds discussed above is methyl and generally in this class of compounds the $R^1$ is ethyl and propyl compounds are superior herbicides to their $R^1$ is methyl homologs. The compound of Formula I hereinbelow wherein R is H is not only an excellent herbicide against grasses it is also surprisingly more phytotoxic than both the $R^1$ is ethyl and $R^1$ is propyl homologs. The 3-alkanoyl (i.e., Formula I, R is $-\overset{O}{\overset{\parallel}{C}}R2$) derivative of this compound are also excellent herbicides.

The present compounds exhibit both pre-emergence and post-emergence phytotoxicity with respect to grasses and exhibit excellent especially post-emergence herbicidal activity against Bermudagrass, foxtail, crabgrass, volunteer corn, volunteer sorghum, barnyardgrass, broad-leaf signalgrass, goosegrass, red rice, sprangletop, seedling Johnsongrass and Rhizome Johnsongrass. The compounds of Formula I, and their salts, are very useful for controlling grasses and are especially useful for controlling grassy weeds in sugar beet crops. The outstanding herbicidal activity possessed by the present compounds rarely exists and cannot be predicted beforehand.

The compounds of the present invention are represented by the following formula:

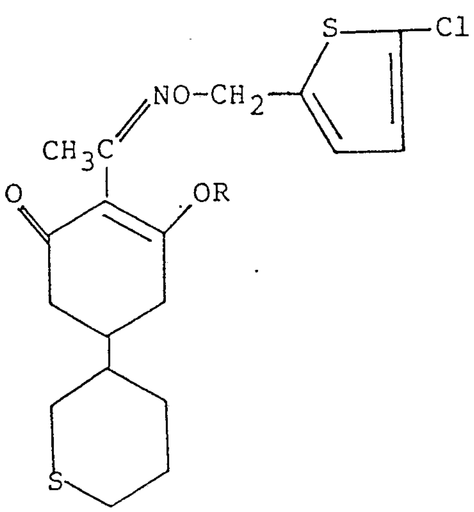

(I)

wherein R is hydrogen, a cation yielding a compatible salt or

$-\overset{O}{\overset{\parallel}{C}}R^2$ wherein $R^2$ is alkyl having 1 to 10 carbon atoms, preferably 1 to 7 carbon atoms.

As is well recognized, compounds of the nature of Formula (I) exist as tautomers. The compounds also have two asymmetric carbon atoms and can also exist as optical isomers. The above formula is intended to encompass the respective tautomeric forms as well as the individual optical isomers as well as mixtures thereof and the respective tautomers and optical isomers as well as mixtures thereof are encompassed within the invention.

The invention also provides a process for inhibiting the growth of axillary buds plants which comprises applying to said tobacco plants or their growth medium an amount effective to inhibit the growth of said axillary buds of a growth control agent selected from compounds of Formula (I) and mixtures of such compounds.

In a further aspect the invention provides a tobacco axillary bud controlling composition comprising a compatible carrier and a tobacco axillary bud controlling effective amount of the compound(s) of Formula (I) or mixtures thereof.

In still a further aspect the invention provides a herbicidal composition comprising a compatible carrier and a herbicidally effective amount of the compound(s) of the invention or mixtures thereof.

The present invention also provides a method for preventing or controlling the growth of unwanted grassy vegetation, which comprises treating the growth medium and/or the foliage of such vegetation with a herbicidally effective amount of the compound(s) of the invention or mixtures thereof.

The present invention also provides a method for regulating plant growth which comprises treating the growth medium and/or the foliage of such vegetation with a plant growth regulating effective amount of the compound(s) of the invention or mixtures thereof, effective to alter the normal growth pattern of said plants.

There are also disclosed herein chemical intermediates and processes for preparing the compounds of the invention.

The invention will be further described hereinbelow.

In general the compounds where R is alkanoyl (i.e., $-\overset{O}{\overset{\|}{C}}R^2$) are more effective for controlling tobacco suckers than the corresponding 3-hydroxy compound (i.e., R is hydrogen) or its salts, though both are highly active for this purpose.

The preferred compounds for controlling tobacco suckers (i.e., axillary buds) are those wherein $R^2$ is alkyl having 1 to 7 carbon atoms, more preferably 1 to 3 carbon atoms and most preferably methyl, ethyl, or n-propyl.

Typically, the compounds are applied at application rates of 0.1-3.2 kilograms per hectare ("Kg/ha") preferably 0.4-2 Kg/ha. Best results are typically obtained using application rates of about from 0.8-1.6 Kg/ha. Mixtures of the compounds of Formula I can also be used. Optimum results may vary with the particular compound or compounds of Formula I used and the particular species of tobacco but can be obtained by routine experimentation. At high dosage rates (e.g., 5 kg/ha or above) certain of the compounds exhibit phytotoxicity with respect to certain broadleaf plants as well as grasses. Thus, high dosage rates should be avoided.

As noted above, it is conventional to remove the terminal growing meristem of the tobacco plant in order to optimize the number and quality of usable leaves. In accordance, with the practice of the present invention, it is preferable to apply the compounds of Formula I or their salts within two days, more preferably a day after removal of the terminal growing meristem. The compounds can be applied directly to the plants or to the soil. Most typically the compound is applied as a solution or liquid emulsion.

Although in theory the compounds can be applied undiluted, in actual practice they are generally applied as a composition or formulation, comprising an effective amount of the compound(s) of Formula I or their salts and an acceptable carrier. An acceptable or compatible carrier (agriculturally acceptable carrier) is one which does not significantly adversely affect the desired biological effect achieved by the active compounds, save to dilute it. The compositions can be prepared, both for direct application and also a concentrate designed for dilution prior to application. The carrier can be a solid, liquid, or aerosol. The actual compositions can take the form of granules, powders, dusts, solutions, emulsions, slurries, aerosols, and the like.

Suitable solid carriers which can be used include, for example, natural clays (such as kaolin, attapulgite, montmorillonite, etc.), talcs, pyrophyllite, diatomaceous silica, synthetic fine silica, calcium aluminosilicate, tricalcium phosphate, and the like. Also, organic materials, such as, for example, walnut shell flour, cotton-seed hulls, wheat flour, wood flour, wood bark flour, and the like can also be used as carriers. Suitable liquid diluents or carriers which can be used include, for example, water, organic solvents (e.g., hydrocarbons such as benzene, toluene, dimethylsulfoxide, kerosene, diesel fuel, fuel oil, petroleum naphtha, etc.), and the like. Suitable aerosol carriers which can be used include conventional aerosol carriers such as halogenated alkanes, etc.

The composition can also contain various promoters and surface-active agents which enhance the rate of transport of the active compound into the plant tissue such as, for example, organic solvents, wetting agents and oils.

The composition can also contain various compatible adjuvants, stabilizers, conditioners, insecticides, fungicides.

One convenient concentrate formulation which can be used comprises 20-30 by weight percent of the compound(s) of Formula I or salts thereof, of the invention, 2-4 by weight percent of an emulsifier, for example, alkylarylsulfonates, e.g., calcium alkylbenzene sulfonates, octylphenolethoxylate, polyoxyethylenealkylaryl, etc., or mixtures thereof, and about 66-76% organic solvent, for example, petroleum hydrocarbon and aromatics, e.g., xylene, kerosene, etc. For example, one suitable concentrate formulation which can be used comprises about 25 wt % of the active compound; about 72 wt % of a xylene base solvent sold under the Trademark "HiSol 10" by Ashland Oil Company, as the solvent and about 3 wt % of mixture of alkylarylsulfonates and polyoxyethylenealkylaryl sold under the Trademark "Atlox 3454F" as the emulsifier.

The concentrate can be mixed with water, optionally containing a crop oil, prior to application and applied as

a water emulsion, optionally containing about 0.125-2 wt % of a crop oil, for example, soybean oils, and paraffinic oils and olefinic oils and/or surfactant. Conveniently, the composition is applied as a water emulsion spray containing from 0.01-0.32 wt %, preferably 0.04-0.16 wt % of the compound(s) of the invention; from 0.001-0.0625 wt % of an emulsifier; from 0.08-3.3 wt % of an organic solvent and from 95-99 wt % water and optionally 0.125-2 wt % crop oil. The spray composition can be conveniently prepared by mixing the concentrate formulation with from 1/4-1/2 the desired amount of water. Then admixing the crop oil, if used, and then adding the remaining amount of water. If no crop oil or additional surfactant is used, then the water and concentrate formulation are simply admixed together.

The compounds of Formula (I) and their salts also exhibit both pre- and post-emergent herbicidal activity and exhibit especially good herbicidal activity against grasses. The compounds exhibit especially good phytotoxicity against foxtail, Bermudagrass, crabgrass, rhizome, Johnsongrass and volunteer corn. These weed species are generally very difficult to control and hence, the present compounds provide a significant advantage which respect to the control of such weeds.

Generally, for post-emergent applications, the herbicidal compounds are applied directly to the foliage or other plant parts. For pre-emergence applications, the herbicidal compounds are applied to the growth medium, or prospective growth medium, for the plant. The optimum amount of the herbicidal compound or composition will vary with the particular plant species, and the extent of plant growth, if any, and the particular part of the plant which is contacted and the extent of contact. The optimum dosage can also vary with the general location, or environment (e.g., sheltered areas such as greenhouses compared to exposed areas such as fields), and type and degree of control desired. Generally, for both pre- and post-emergent control, the present compounds are applied at rates of from 0.02 to 60 kg/ha, preferably from 0.02 to 10 kg/ha.

Also, although in theory the compounds can be applied undiluted, in actual practice they are generally applied as a herbicidal composition or formulation comprising an effective amount of the compound(s) and an acceptable carrier. As noted above, acceptable or compatible carrier (agriculturally acceptable carrier) is one which does not significantly adversely affect the desired biological effect achieved by the active compounds, save to dilute it. Typically, the composition contains from 0.05 to 95% by weight of the compound of Formula (I) or mixtures thereof. Concentrates can also be made having high concentrations designed for dilution prior to application. The carrier can be a solid, liquid, or aerosol. The actual compositions can take the form of granules, powders, dusts, solutions, emulsions, slurries, aerosols, and the like.

The same types of carrier materials as described above with respect to the composition for controlling tobacco suckers can also be used for the herbicidal compositions.

The composition can also contain various promoters and surface-active agents which enhance the rate of transport of the active compound into the plant tissue such as, for example, organic solvents, wetting agents and oils, and in the case of compositions designed for pre-emergence application agents which reduce the leachability of the compound or otherwise enhance soil stability. Crop oils, such as, for example, soybean oils, paraffin oils and olefinic oils, are especially advantageous as carriers or additives in that they enhance phytotoxicity. Various other adjuvants available to enhance the phytotoxicity of oximenychlohexanedione class of herbicides can also be advantageously used.

The composition can also contain various compatible adjuvants, stabilizers, conditioners, insecticides, fungicides, and if desired, other herbicidally active compounds.

One convenient concentrate formulation which can be used comprises 23-27% by weight of the active herbicide of the invention, 2 to 4% by weight of an emulsifier, for example, calcium alkylbenzene sulfonates, octylphenolethoxylate, etc., or mixtures thereof, and about 70-75% organic solvent, for example, xylene, etc. The concentrate is mixed with water and preferably a crop oil prior to application and applied as a water emulsion containing from 0.5 to 2% of a crop oil, for example, soybean oils, and paraffinic oils and olefinic oils. Conveniently the herbicide is applied as water emulsion containing from 0.02-0.6 wt. %, preferably 0.07-0.15 wt. % of the herbicide, of the invention; from 0.001-0.01 wt. % of an emulsifier; from 0.08-2.5 wt. % of an organic solvent and from 95 to 99 wt. % water. Preferably, the composition also contains from 0.25 to 2 wt. % of a crop oil. The application composition can be conveniently prepared by mixing the concentrate formulation with from 1/4 to 1/2 the desired amount of water. Then admixing the crop oil and then adding the remaining amount of water. Mixtures of different crop oils can also be used. If no crop oil is used, then the water and concentrate formulation are simply admixed together.

The compound of Formula I can be conveniently prepared by the following schematically represented process:

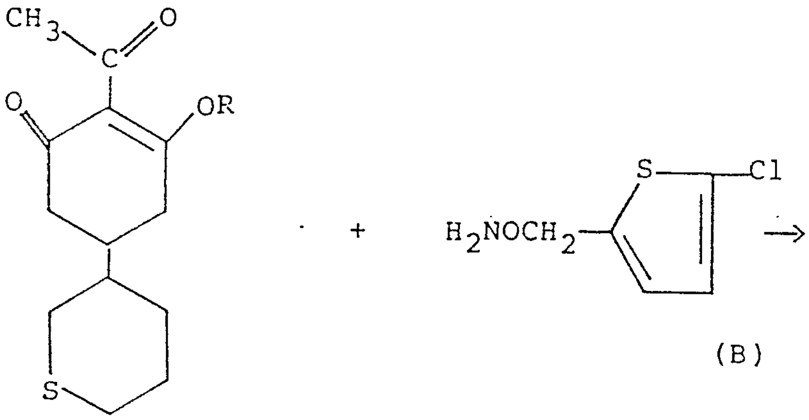

(B) (I)

(A)

wherein R is as defined hereinabove.

This process can be conveniently effected by contacting Compound (A) with (5-chlorothien-2-yl)methoxyamine (B), under reactive conditions preferably in an inert organic solvent.

Typically, this process is conducted at temperatures in the range of from 0-80° C, preferably from 20-40° C, for from 1-48 hours, preferably from 4-12 hours, using from 1-2, preferably 1.05-1.2 moles of (5-chlorothien-2-yl)methoxyamine (B) per mole of Compound (A). Suitable inert organic solvents which can be used include, for example, lower alkanols, e.g., methanol, ethanol; ethers, e.g., ethyl ether; methylene chloride. Two-phase water and immiscible organic solvent (e.g., hexane), and mixtures thereof can also be used as the reaction medium.

(5-chlorothien-2-yl)methoxyamine is a known compound and can be prepared via known procedures, such as, for example, described in my prior U.S. Patent No. 4,432,786. The (5-chlorothien-2-yl)methoxyamine reactant can be conveniently provided by neutralizing its hydrochloride salt in situ with an alkali metal alkoxide. The starting materials of Formula (A) wherein R is hydrogen is also a known compound and can be prepared via the general procedure described in U.S. Patent Nos. 4,624,696 and 4,596,877.

The compound of Formula A wherein R is alkanoyl can be conveniently prepared by acylating the corresponding 3-hydroxy compound:

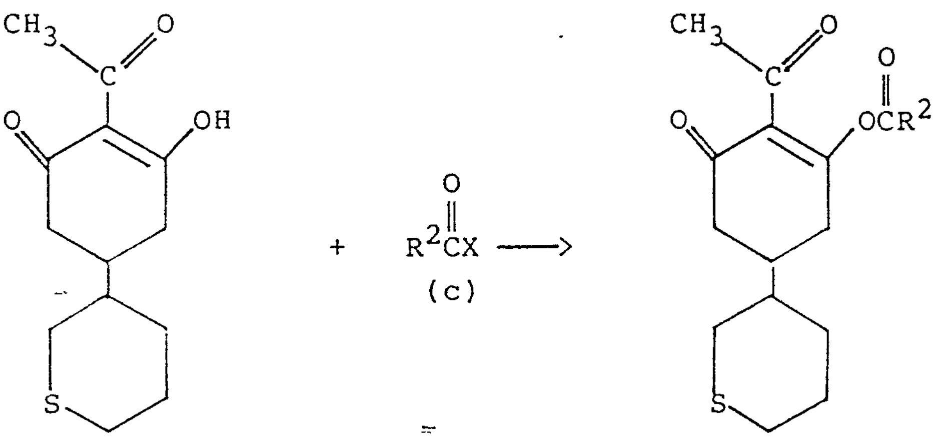

(c)

(A') (A'')

wherein $R^2$ is as defined hereinabove.

This process can be effected by contacting Compound A′ with the appropriate acyl halide under reactive conditions preferably in an inert organic solvent and preferably in the presence of a scavenger base.

Typically this process is conducted at temperatures in the range of from -78 to 100° C, preferably 0 to 25° C for from 1 to 48 hours using from 1 to 5 moles, preferably 1 to 1.2 moles of acyl halide (c) per mole of Compound A′. Where a scavenger base is used typically from 1 to 5, preferably 1 to 1.2 moles of scavenger

base is used per mole of Compound A′. Suitable scavenger bases which can be used include, for example, triethylamine, pyridine, methylpyridine, 2,4-lutidine, and the like. Suitable solvents which can be used include, for example methylene chloride, chloroform, ethyl acetate, acetonitrile, diethyl ether, tetrahydrofuran, and the like.

The R is alkanoyl compounds of Formula I can also be prepared by acylating the 3-hydroxy analog of Formula I wherein R is hydrogen. For example, the acylation can be effected using the appropriate acyl halide using substantially the same conditions as described above with respect to the acylation of Compound A′. Other acylating agents could also be used in either case such as, for example, acyl anhydrides. The compounds of Formula A′ and the 3-hydroxy compound of Formula I can be prepared via the procedure described in U.S. Patent No. 4,624,696.

The compatible salts of the compounds of Formula (I) can be prepared by conventional procedures, for example, via the reaction of the compound of Formula I, wherein R is hydrogen, with a base, such as, for example, sodium hydroxide, potassium hydroxide and the like, having the desired cation. Additional variations in the salt cation can also be effected via ion exchange with an ion exchange resin having the desired cation.

The term "compatible salts" refers to salts which do not significantly adversely alter the herbicidal or tobacco sucker control, as the case may be, properties of the parent compound. Suitable salts include cation salts such as, for example, the cation salts of lithium, sodium, potassium, alkali earth metals, copper, zinc, ammonia, quaternary ammonium salts, and the like.

General Process Conditions

The reaction product can be recovered from its reaction product mixture by any suitable separation and purification procedure, such as, for example, chromatography. Suitable separation and purification procedures are, for example, illustrated in the Examples set forth hereinbelow.

Generally, the reactions described above are conducted as liquid phase reaction and hence pressure is generally not significant except as it affects temperature (boiling point) where reactions are conducted at reflux. Therefore, these reactions are generally conducted at pressures of about from 300-3000 mm of mercury and conveniently are conducted at about atmospheric or ambient pressure.

It should also be appreciated that where typical or preferred process conditions (e.g., reaction temperatures, times, mole ratios of reactants, solvents, etc.) have been given, that other process conditions could also be used. Optimum reaction conditions (e.g., temperature, reaction time, mol ratios, solvents, etc.) may vary with the particular reagents or organic solvents used but can be determined by routine optimization procedures.

Definitions

As used herein the following terms have the following meanings unless expressly stated to the contrary:

The term "alkyl" includes both straight chain and branched chain alkyl groups.

The term "alkylidene" includes both straight chain and branched chain alkylidene groups and includes, for example, groups having the formula

```
-(CH2)2-; -CH2 CH -; - CH3  ; -(CH2)3; and the like.
               |       |
               CH      CH
```

The term "room temperature" or "ambient temperature" refers to about 20-25°C.

A further understanding of the invention can be had in the following non-limiting Preparation(s) and Example(s). Wherein, unless expressly stated to the contrary, all temperatures and temperature ranges refer to the Centigrade system and the term "ambient" or "room temperature" refers to about 20-25°C. The term "percent" or "%" refers to weight percent and the term "mole" or "moles" refers to gram moles. The term "equivalent" refers to a quantity of reagent equal in moles, to the moles of the preceding or succeeding reactant recited in that example in terms of finite moles or finite weight or volume.

## EXAMPLES

### Example 1

2-[1-[(5-chlorothien-2-yl)methoxyimino]ethyl]-3-hydroxy-5-(tetrahydro-2H-thiopyran-3-yl)-cyclohex-2-en-1-one

A solution containing 5.1 g (0.020 mol) of 2-acetyl-3-hydroxy-5-(tetrahydro-2H-thiopyran-3-yl)-cyclohex-2-en-1-one and 3.4 g (0.021 mol) of (5-chlorothien-2-yl)methoxyamine in 200 ml of ethanol was stirred overnight (about 14 hours) at room temprature and then evaporated to remove ethanol. The concentrate was diluted with 150 ml of methylene chloride and then washed with 100 ml of water and then with 100 ml of saturated aqueous sodium chloride solution. The washed methylene chloride solution was dried over

anhydrous magnesium sulfate and then evaporated to dryness under reduced pressure to afford a white solid. Recrystallation of this solid from absolute ethanol afforded 7.6 g of the title compound, m.p. 154-155° C.

## Example 2

### Sodium 2-[1-[(5-chlorothien-2-yl)methoxyimino]ethyl]-3-oxo-5-(tetrahydro-2H-thiopyran-3-yl)-cyclohex-1-en-1-olate

This example illustrates a procedure which can be used to prepare the title compound.

A solution containing 0.01 mol of sodium hydroxide dissolved in 2 ml of water is added to a solution containing 0.01 mol of 2-[1-[(5-chlorothien-2-yl)methoxyimino]ethyl]-3-hydroxy-5-(tetrahydro-2H-thiopyran-3-yl)-cyclohex-2-en-1-one at room temperature. After the reaction is completed, the solvents are evaporated off under vacuum affording the 1-hydroxy sodium salt of 2-[1-[(5-chlorothien-2-yl)methoxyimino]ethyl]-3-hydroxy-5-(tetrahydro-2H-thiopyran-3-yl)-cyclohex-2-en-1-one.

## Example 3

### 3-Acetyloxy-2-[1-[(5-chlorothien-2-yl)methoxyimino]ethyl]-5-(tetrahydro-2H-thiopyran-3-yl)-cyclohex-2-en-1-one

In this example, 0.52 g (5.1 mmol) of triethylamine was admixed to a solution containing 1.46 g (3.7 mmol) of 2-[1-[(5-chlorothien-2-yl)methoxyimino]ethyl]-3-hydroxy-5-(tetrahydro-2H-thiopyran-3-yl)-cyclohex-2-en-1-one in 30 ml of methylene chloride at room temperature (about 20-25° C) followed by the dropwise addition of 0.35 g (4.4 mmol) of acetyl chloride. The mixture was stirred at room temperature overnight (about 14 hr) and then washed with saturated aqueous sodium bicarbonate solution, followed by saturated sodium chloride solution. The washed extract was dried over anhydrous magnesium sulfate and concentrated under reduced pressure. The product was purified by column chromatography on silica gel to afford 1.0 g of the title compound as a pale yellow oil; IR (neat) 1775 $cm^{-1}$; $^1H$ NMR spectrum (90 MHz $CDCl_3$) w 1.00-2.60 (m, 14H), 1.93 (s, 3H), 2.00 (s, 3H), 5.07 (s, 2H) 6.73 (s, 2H).

Similarly, by applying the above procedure using the appropriate acyl chloride the following compounds can be prepared:

3-propionyloxy-2-[1-[(5-chlorothien-2-yl)methoxyimino]ethyl]-5-(tetrahydro-2H-thiopyran-3-yl)-cyclohex-2-en-1-one;

3-butyryloxy-2-[1-[(5-chlorothien-2-yl)methoxyimino]ethyl]-5-(tetrahydro-2H-thiopyran-3-yl)-cyclohex-2-en-1-one;

3-isobutyryloxy-2-[1-(5-chlorothien-2-yl)methoxyimino]ethyl]-5-(tetrahydro-2H-thiopyran-3-yl)-cyclohex-2-en-1-one;

3-valeryloxy-2-[1-[(5-chlorothien-2-yl)methoxyimino]ethyl]-5-(tetrahydro-2H-thiopyran-3-yl)-cyclohex-2-en-1-one;

3-isovaleryloxy-2-[1-[(5-chlorothien-2-yl)methoxyimino]ethyl]-5-(tetrahydro-2H-thiopyran-3-yl)-cyclohex-2-en-1-one;

3-pivaloyloxy-2-[1-[(5-chlorothien-2-yl)methoxyimino]ethyl]-5-(tetrahydro-2H-thiopyran-3-yl)-cyclohex-2-en-1-one;

3-hexanoyloxy-2-[1-[(5-chlorothien-2-yl)methoxyimino]ethyl]-5-(tetrahydro-2H-thiopyran-3-yl)-cyclohex-2-en-1-one;

3-heptanoyloxy-2-[1-[(5-chlorothien-2-yl)methoxyimino]ethyl]-5-(tetrahydro-2H-thiopyran-3-yl)-cyclohex-2-en-1-one; and

3-(2-ethylhexanoyloxy-2-[1-[(5-chlorothien-2-yl)methoxyimino]ethyl]-5-(tetrahydro-2H-thiopyran-3-yl)cyclohex-2-en-1-one.

## Example 4

### 2-Acetyl-3-acetyloxy-5-(tetrahydro-2H-thiopyran-3-yl)-cyclohex-2-en-1-one

The title compound can be prepared by the following procedure:

0.82 g (10.5 mmol) of acetyl chloride is added dropwise to a stirred solution of 2.54 g (10.0 mmol) of

2-acetyl-3-hydroxy-5-(tetrahydro-2H-thiopyran-3-yl)-cyclohex-2-en-1-one and 1.06 g (10.5 mmol) of triethylamine in 50 ml of dichloromethane. The mixture is stirred overnight at room temperature, and then is poured into 100 ml of ice-cold water. The organic layer is separated and washed with 20 ml of dilute sodium bicarbonate solution, followed by 50 ml of saturated sodium chloride solution. The washed extract is then dried over anhydrous magnesium sulfate and concentrated under reduced pressure. The product can be purified by column chromatography employing tetrahydrofuran-hexane (1:2 by volume) as eluent to afford the title compound.

## Example 5

### 3-Acetyloxy-2-[1-[(5-chlorothien-2-yl)methoxyimino]ethyl-5-(tetrahydro-2H-thiopyran-3-yl)-cyclohex-2-en-1-one

The title compound can be prepared by the following procedure:
0.34 g (2.0 mmol) of (5-chlorothien-2-yl)methoxyamine is added to a stirred solution of 0.59 g (2.0 mmol) of 2-acetyl-3-acetyloxy-5-(tetrahydro-2H-thiopyran-3-yl)-cyclohex-2-en-1-one in 10 ml of absolute ethanol. After stirring overnight at room temperature, the reaction mixture is concentrated under reduced pressure. The resulting residue is diluted with 25 ml of dichloromethane and washed twice with 10 ml of saturated sodium chloride solution. The washed extract is then dried over anhydrous magnesium sulfate and evaporated under reduced pressure. The product can then be purified by column chromatography employing tetrahydrofuran-hexane (1:2 by volume) as eluent to afford the title compound.

## Example 6

In this example, the compounds identified in Table A hereinbelow were tested for their ability to control (retard or prevent) lateral axillary buds in tobacco plants.

These tests were conducted using the following procedure.

The results are reported as an average of the replicates used in each test. The results of these tests and the dosage rates, evaluation times, etc., used are indicated in the following tables.

The tobacco plants used in these studies were topped approximately 2 hours before chemical treatment. Topping was done by removing all but 8 fully expanded leaves. Any plants that showed premature yellowing of leaves or bending of stems were discarded. All the pots were labeled and 5 replicates were used for each treatment.

A stock solution was prepared by dissolving the respective compound in acetone containing a non-ionic emulsifier. The stock solution was diluted with deionized water containing 0.625 g of emulsifier per liter of water, to the desired spray solution concentrations. The exact concentration and species of tobacco plant used for testing are indicated in the individual data charts.

Each treatment was applied until run-off to individual plants with a pressure atomizer to ensure good coverage of various plant parts with the chemical solution. The only exception to this rule was the experiment on translocation studies (Test No. 13439) where the compound was either painted on the leaf with a fine paint brush or applied as a soill drench treatment. Each plant was sprayed with 7-10 ml of solution. After spraying, the plants were maintained in a greenhouse at 21-26°C for up to 6 weeks depending on the experiment. All plants were fertilized once a week with a standard garden fertilizer. Readings were taken on percent sucker control at different time intervals. This was accomplished by making visual assessment of bud growth in treated plants compared to bud growth in untreated plants, also referred to as checks. Data was recorded as percent bud inhibition (sucker control), with 0 = no inhibition of buds and 100 = total inhibition buds, compared to untreated checks. The sucker development of the top 3 nodes was referred to as primary suckers whereas the sucker development on lower nodes was referred to as secondary suckers. (In general, primary suckers are substantially more detrimental to tobacco production than secondary suckers.)

TABLE A

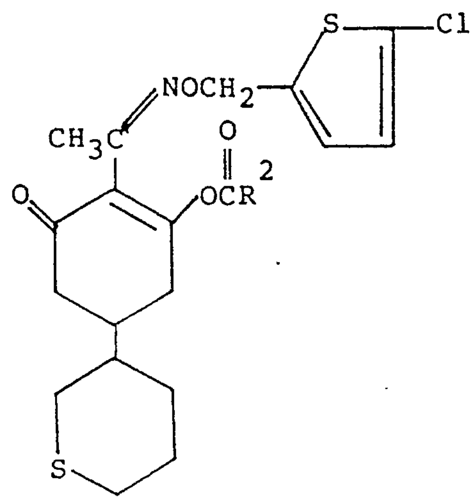

| Compound No. | $R^2$ |
|---|---|
| 1 | $CH_3$ |
| 2 | $CH_3CH_2-$ |
| 3 | $CH_3(CH_2)_2-$ |
| 4 | $CH_3(CH_2)_3-$ |
| 5 | $CH_3(CH_2)_4-$ |

TABLE 1

Tobacco Sucker Control (Var. Glurk)[1]

Three Weeks After Treatment[3]

| Compound | Dosage[2] Concentration mg/l | Percent Primary Sucker Inhibition |
|---|---|---|
| 1 | 100 | 93 |
| 1 | 50 | 80 |
| 1 | 25 | 30 |
| 1 | 12.5 | 0 |
| 2 | 100 | 91 |
| 2 | 50 | 73 |
| 2 | 25 | 33 |
| 2 | 12.5 | 0 |
| 3 | 100 | 98 |
| 3 | 50 | 85 |
| 3 | 25 | 20 |
| 3 | 12.5 | 0 |
| 4 | 100 | 94 |
| 4 | 50 | 58 |
| 4 | 25 | 28 |
| 4 | 12.5 | 0 |
| 5 | 100 | 97 |
| 5 | 50 | 71 |
| 5 | 25 | 0 |
| 5 | 12.5 | 0 |

Six Weeks After Treatment[3]

| Compound | Dosage[2] Concentration mg/l | Percent Primary Sucker Inhibition Top two Nodes | Number of Secondary Suckers[4] |
|---|---|---|---|
| 1 | 200 | 99 | 0.2 |
| 1 | 100 | 99 | 2.5 |
| 1 | 50 | 98 | 5.2 |
| 1 | 25 | 98 | 6.0 |
| 2 | 100 | 50 | 1.5 |
| 2 | 50 | 35 | 1.5 |
| 2 | 25 | 28 | 1.3 |
| 2 | 12.5 | 8 | 3.0 |
| 3 | 100 | 80 | 5.5 |
| 3 | 50 | 40 | 1.8 |
| 3 | 25 | 20 | 1.0 |
| 3 | 12.5 | 5 | 2.3 |
| 4 | 100 | 61 | 1.5 |
| 4 | 50 | 35 | 0.8 |
| 4 | 25 | 23 | 1.0 |
| 4 | 12.5 | 3 | 1.8 |
| 5 | 100 | 78 | 2.8 |
| 5 | 50 | 25 | 0.5 |
| 5 | 25 | 0 | 0.8 |
| 5 | 12.5 | 0 | 2.0 |

1] Var. Glurk: Nicotiana tabacum (At the spray volume and area used in the tests 1000 mg/l equals an application rate approximately 1 Kg/ha)
2] Concentration of test compound in spray solution
3] Weeks after spraying of tobacco plants with test compound
4] i.e., Axillary buds at lower nodes

## Example 7

In this example, the Compound No. 1 was tested side-by-side with its 3-hydroxy analog (Compound No. 6) for tobacco sucker control. These tests were conducted following the same general procedure as described in Example 3 hereinabove using the dosage concentrations given in the tables hereinbelow.

TABLE 2

Tobacco Sucker Control (Var. Glurk)[1]

Three Weeks After Treatment[3]

| Compound | Dosage[2] Concentration mg/l | Percent Bud Inhibition Primary Suckers Var. Glurk[1] |
|---|---|---|
| 1[4] | 200 | 100 |
| 1 | 100 | 100 |
| 1 | 50 | 99 |
| 1 | 25 | 92 |
| 6[4] | 200 | 100 |
| 6 | 100 | 100 |
| 6 | 50 | 100 |
| 6 | 25 | 38 |
| Check | - | 0 |

1] Variety Glurk: Nicotiana tabacum
2] Concentration of test compound in spray solution; at volume and area used, 1000 mg/l equals about 1 Kg/ha
3] Weeks after spraying of tobacco plants with test compound
4] Compound No. 6 is 3-hydroxy-2-[1-(5-chlorothien-2-ylmethoxyimino)ethyl]-5-(tetrahydro-2H-thiopyran-3-yl)-cyclohex-2-en-1-one

As can be seen from the above test, both compounds exhibited excellent sucker oontrol at high to moderate dosage rates, but at the low 25 mg/l application rate Compound 6 exhibited only poor (i.e., 38%) sucker control whereas Compound 1 still exhibited very good sucker control (i.e., 92%).

## Example 48

In this example, the title compound of Example 1, i.e., 2-[1-[(5-chlorothien-2-yl)methoxyimino]ethyl]-3-hydroxy-5-(tetrahydro-2H-thiopyran-3-yl)-cyclohex-2-en-1-one (Compound No. 6) and its $R^1$ is ethyl and propyl homologs, i.e., 2-[1-[(5-chlorothien-2-yl)methoxyimino]propyl]-3-hydroxy-5-(tetrahydro-2H-thiopyran-3-yl)-cyclohex-2-en-1-one (C-1) and 2-[1-[(5-chlorothien-2-yl)methoxyimino]butyl]-3-hydroxy-5-(tetrahydro-2H-thiopyran-3-yl)-cyclohex-2-en-1-one (C-2); were tested, side-by-side using the procedures described hereinbelow, for pre-emergent and post-emergent phytotoxic activity against a variety of grasses and broad-leaf plants.

### Pre-Emergent Herbicide Test

Pre-emergence herbicidal activity was determined in the following manner.

Test solutions of the respective compounds were prepared as follows:

355.5 mg of test compound was dissolved in 15 ml of acetone. 2 ml of acetone containing 110 mg of a nonionic surfactant was added to the solution. 12 ml of this stock solution was then added to 47.7 ml of water which contained the same nonionic surfactant at a concentration of 625 mg/l. Dilutions were made as needed to arrive at the desired spray concentrations.

Seeds of the test vegetation were planted in a pot of soil and the test solution was sprayed uniformly onto the soil surface at the test compound dosage indicated in Table 3. Three replicate pots were used per species and rate. The pots were watered and placed in a greenhouse. The pots were watered intermittently and observed for seedling emergence, health of emerging seedlings, etc., for a 3-week period. At the end of this period, the herbicidal effectiveness of the compound was rated based on the physiological observations. A 0-to-100 scale was used, 0 representing no phytotoxicity, 100 representing complete kill. The results of these tests are expressed as an average of the results for the three replicates and are summarized in Table 3.

## Post-Emergent Herbicidal Test

The test compound was formulated in the same manner as described above for the pre-emergent test. This formulation was uniformly sprayed on 2 similar pots containing plants 2 to 3" (5 to 7.5cm) tall (except wild oats, soybean and barnyardgrass which were 3 to 4" (7.5 to 10 cm) tall) (approximately 15 to 25 plants per pot) at the test compound dose indicated in Table 4. After the plants had dried, they were placed in a greenhouse and then watered intermittently at their bases as needed. The plants were observed periodically for phytotoxic effects and physiological and morphological responses to the treatment. Three replicate pots were used per species and test rate. After 3 weeks, the herbicidal effectiveness of the compound was rated based on these observations. A 0-to-100 scale was used, 0 representing no phytotoxicity, 100 representing complete kill. The results of these tests are expressed as an average of the results for the replicates and are summarized in Table 4.

In the aforedescribed tests, phytotoxicities below about 20-30% are generally not considered meaningful because generally the plant can grow out of this amount of injury.

The compounds tested are identified by structured formats in Table B, hereinbelow.

Table B

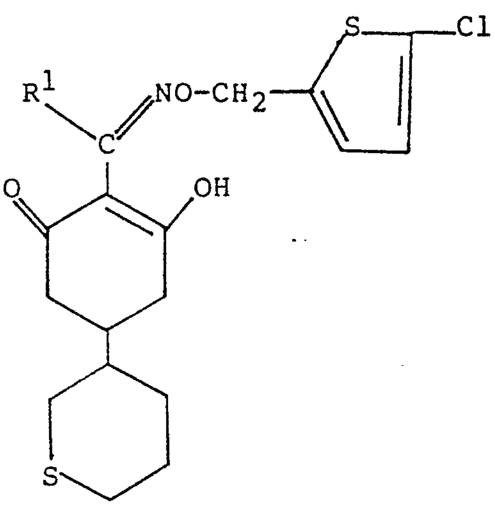

| Compound Test No. | $R^1$ |
|---|---|
| 6 | $-CH_3$ |
| C-1* | $-CH_2CH_3$ |
| C-2* | $-CH_2CH_2CH_3$ |

*C-numbers indicate comparison compounds

TABLE 3

Pre-Emergence Herbicidal Activity - Percent Control [1]

Low Dosage Tests

| Compound No. | Dosage q/cm² | Grasses, % Phytotoxicity: Crabgrass | Barnyard Grass** | Wild Oats | Rhizome Johnson-Grass*** | Yellow Foxtail | Cheat Grass | Blackgrass | Goosegrass |
|---|---|---|---|---|---|---|---|---|---|
| 6 | 0.70 | 100 | 100 | 65 | - | 100 | 0 | 100 | 100 |
| 6 | 0.28 | 100 | 95 | 0 | - | 75 | 0 | 65 | 99 |
| 6 | 0.11 | 15 | 20 | 0 | - | 15 | 0 | 0 | 57 |
| 6 | 0.04 | 0 | 0 | 0 | - | 0 | 0 | 0 | 0 |
| C-1 | 0.70 | 99 | 100 | 45 | - | 100 | 0 | 97 | 100 |
| C-1 | 0.28 | 72 | 60 | 0 | - | 90 | 0 | 30 | 90 |
| C-1 | 0.11 | 0 | 0 | 0 | - | 0 | 0 | 0 | 30 |
| C-1 | 0.04 | 0 | 0 | 0 | - | 0 | 0 | 0 | 0 |
| C-2 | 0.70 | 100 | 100 | 15 | - | 100 | 0 | 85 | 100 |
| C-2 | 0.28 | 67 | 70 | 0 | - | 55 | 0 | 25 | 79 |
| C-2 | 0.11 | 0 | 0 | 0 | - | 0 | 0 | 0 | 0 |
| C-2 | 0.04 | 0 | 0 | 0 | - | 0 | 0 | 0 | 0 |
| Check | | | | | | | | | |

* q/cm² = micrograms/square centimeter.

** California Barnyardgrass.

*** Not tested.

[1] Reported as average of four replicates.

TABLE 3 (Cont'd)

| Compound No. | Dosage q/cm²* | Grasses % Phytotoxicity | | | | | | |
|---|---|---|---|---|---|---|---|---|
| | | Sprangletop | Italian Rye Grass | Fall Panicum | Proso Millet | Barley | Rice | Sorghum |
| 6 | 0.70 | 100 | 97 | - | 100 | 0 | 70 | 100 |
| 6 | 0.28 | 0 | 0 | - | 45 | 0 | 0 | 40 |
| 6 | 0.11 | 0 | 0 | - | 0 | 0 | 0 | 0 |
| 6 | 0.05 | 0 | 0 | - | 0 | 0 | 0 | 0 |
| C-1 | 0.70 | 90 | 100 | - | 100 | 0 | 50 | 87 |
| C-1 | 0.28 | 15 | 0 | - | 45 | 0 | 0 | 0 |
| C-1 | 0.11 | 0 | 0 | - | 0 | 0 | 0 | 0 |
| C-1 | 0.05 | 0 | 0 | - | 0 | 0 | 0 | 0 |
| C-2 | 0.70 | 100 | 100 | - | 100 | 0 | 0 | 0 |
| C-2 | 0.28 | 0 | 0 | - | 30 | 0 | 0 | 0 |
| C-2 | 0.11 | 0 | 0 | - | 0 | 0 | 0 | 0 |
| C-2 | 0.05 | 0 | 0 | - | 0 | 0 | 0 | 0 |

* $q/cm^2$ = micrograms/square centimeter

TABLE 4

Post-Emergence Herbicidal Activity - Percent Control [1]

Low Dosage Tests

| Compound No. | Dosage g/cm²* | Grasses % Phytotoxicity | | | | | | | |
|---|---|---|---|---|---|---|---|---|---|
| | | Crabgrass | Barnyard Grass** | Wild Oats | Rhizome Johnson-Grass | Yellow Foxtail | Cheat Grass | Blackgrass | Goosegrass |
| 6 | 0.28 | 100 | 100 | 100 | 100 | 99 | 99 | 87 | 100 |
| 6 | 0.11 | 100 | 100 | 92 | 100 | 99 | 92 | 95 | 99 |
| 6 | 0.04 | 96 | 97 | 0 | 92 | 67 | 55 | 45 | 98 |
| 6 | 0.02 | 12 | 10 | 0 | 0 | 0 | 0 | 0 | 10 |
| C-1 | 0.28 | 98 | 100 | 95 | 89 | 98 | 95 | 70 | 98 |
| C-1 | 0.11 | 90 | 80 | 10 | 30 | 92 | 30 | 60 | 85 |
| C-1 | 0.04 | 0 | 0 | 0 | 0 | 90 | 0 | 0 | 0 |
| C-1 | 0.02 | 0 | 0 | 0 | 0 | 0 | 0 | 0 | 0 |
| C-2 | 0.28 | 70 | 100 | 35 | 80 | 96 | 87 | 75 | 95 |
| C-2 | 0.11 | 0 | 100 | 30 | 20 | 92 | 55 | 20 | 85 |
| C-2 | 0.04 | 0 | 82 | 0 | 0 | 0 | 0 | 0 | 10 |
| C-2 | | | | | | | | | |
| Check | 0 | 0 | 0 | 0 | 0 | 0 | 0 | 0 | 0 |

* q/cm² = microgram/square centimeter

** California Barnyardgrass.

[1] Reported as average of three replicates.

TABLE 4 (Cont'd)

| Compound No. | Dosage q/cm²* | Grasses % Phytotoxicity | | | | | |
|---|---|---|---|---|---|---|---|
| | | Sprangletop | Italian Rye Grass | Fall Panicum | Proso Millet | Field Corn** | Sorghum** |
| 6 | 0.28 | 98 | 99 | 99 | 100 | 100 | 100 |
| 6 | 0.11 | 52 | 95 | 97 | 95 | 100 | 99 |
| 6 | 0.04 | 0 | 40 | 90 | 55 | 85 | 60 |
| 6 | 0.02 | 0 | 0 | 10 | 0 | 5 | 0 |
| C-1 | 0.28 | 100 | 100 | 97 | 95 | 95 | 95 |
| C-1 | 0.11 | 25 | 80 | 95 | 30 | 55 | 87 |
| C-1 | 0.04 | 0 | 20 | 30 | 0 | 0 | 35 |
| C-1 | 0.02 | 0 | 0 | 20 | 0 | 0 | 0 |
| C-2 | 0.28 | 75 | 100 | 95 | 96 | 80 | 90 |
| C-2 | 0.11 | 0 | 95 | 90 | 60 | 60 | 55 |
| C-2 | 0.04 | 0 | 20 | 10 | 0 | 0 | 0 |

* $q/cm^2$ = $micrograms/cm^2$.
** Field corn and sorghum presents significant weed problems where crops are rotated.

TABLE 4A

CROPS

Post-Emergence Herbicidal Activity - Percent Control [1]

Low Dosage Tests

| Compound No. | Dosage q/cm$^2$* | Grasses % Phytotoxicity | | | Broadleaf % Phytotoxicity | | |
|---|---|---|---|---|---|---|---|
| | | Barley | Rice | Anza Wheat | Sugar Beet | Cotton | Soybean |
| 6 | 0.28 | 100 | 100 | 85 | 5 | 40 | 57 |
| 6 | 0.11 | 82 | 95 | 70 | 0 | 25 | 40 |
| 6 | 0.04 | 30 | 50 | 0 | 0 | 12 | 30 |
| 6 | 0.02 | 0 | 0 | 0 | 0 | 5 | 20 |
| C-1 | 0.28 | 99 | 96 | 65 | 2 | 7 | 10 |
| C-1 | 0.11 | 95 | 50 | 25 | 0 | 0 | 10 |
| C-1 | 0.04 | 0 | 0 | 0 | 0 | 0 | 0 |
| C-1 | 0.02 | 0 | 0 | 0 | 0 | 0 | 0 |
| C-2 | 0.28 | 99 | 50 | 70 | 0 | 7 | 30 |
| C-2 | 0.11 | 99 | 40 | 60 | 0 | 0 | 15 |
| C-2 | 0.04 | 50 | 0 | 0 | 0 | 0 | 0 |
| C-2 | | | | | | | |
| Check | 0 | 0 | 0 | 0 | 0 | 0 | 0 |

* q/cm$^2$ = microgram/square centimeter.
[1] Reported as average of four replicates.

Based on the results shown in the above tables it can be seen that all three compounds exhibit both pre-emergent and post-emergence phytotoxicity against grasses but are substantially more active as post-emergent herbicides. (This is generally true of the class of oximecyclohexanedione herbicides and the commercial examples of the class of herbicide are primarily used as post-emergent herbicides.) From the post-emergent herbicide results shown in the tables, it can be seen that Compound 6, of the present invention, was more active than comparison Compound C-2 against thirteen of the fourteen weed grasses tested and was as active as Compound C-2 against the fourteenth. Comparison Compound C-1 was more active than Compound C-2, but, nonetheless, Compound 1 was more active than Compound C-1 against ten of the fourteen weed grasses. With respect to the remaining four grasses, Compound 6 was somewhat more active than Compound C-1 but not sufficiently so, that good control could be obtained using the next lower dosage level in the table's compound with the dosage level used form Compound C-1. The only instance where Compound C-1 was more active than Compound 6 was with respect to yellow foxtail at the 0.04 g/$cm^2$ dosage rate. In the case of crabgrass, barnyardgrass, goosegrass, and Fall Panicum a dosage rate of Compound 6 of 0.04 g/$cm^2$ was as effective or more effective than a dosage rate of 0.11 g/$cm^2$ of Compound C-1. In the case of wild oats, rhizome Johnsongrass, cheatgrass, blackgrass, Proso millet, and field corn, a dosage rate of Compound 6 of 0.11 g/$cm^2$ was as effective or more effective than a dosage rate of Compound C-1 of 0.28 g /$cm^2$. The superiority in post-emergent phytotoxicity of Compound 6 was even greater with respect to Compound C-2.

## Example 9

In this example 3-acetyloxy-2-[1-[(5-chlorothien-2-yl)methoxyimino]ethyl]-5-(tetrahydro-2H-thiopyran-3-yl)-cyclohex-2-en-1-one) (Formula I, R = acetyl, Compound No. 1) was tested for post-emergent herbicide activity side-by-side with its $R^1$ is ethyl and propyl homologs, i.e., 3-acetyloxy-2-[1-[(5-chlorothien-2-yl)methoxyimino]propyl]-5-(tetrahydro-2H-thiopyran-3-yl)-cyclohex-2-en-1-one) (C-3) and 3-acetyloxy-2-[1-[(5-chlorothien-2-yl)methoxyimino]butyl]-5-(tetrahydro-2H-thiopyran-3-yl)-cyclohex-2-en-1-one) (C-4). The tests were conducted in the same manner as described in Example 8 hereinbelow using the application rates given in Table 5 hereinabove. The results of these tests expressed as an average of the results of the replicates for a given compound and application rate are summarized in Table 5 hereinbelow. The components are identified by structure in Table C hereinbelow.

Table C

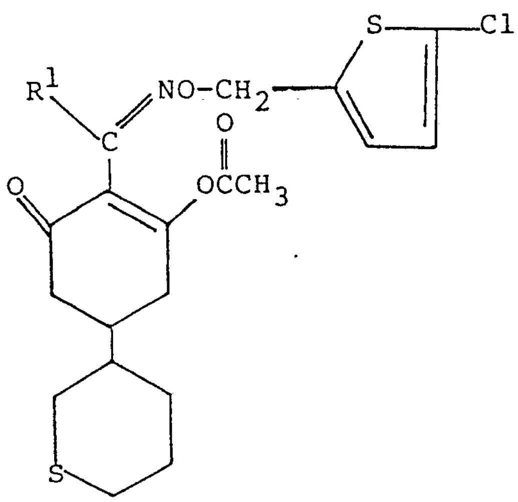

| Compound Test No. | $R^1$ |
|---|---|
| 1 | $-CH_3$ |
| C-3* | $-CH_2CH_3$ |
| C-4* | $-CH_2CH_2CH_3$ |

*C-numbers indicate comparison compounds

TABLE 5

Post-Emergence Herbicidal Activity - Percent Control [1]

Low Dosage Tests

| Compound No. | Dosage q/cm²* | Grasses % Phytotoxicity | | | | | | | |
|---|---|---|---|---|---|---|---|---|---|
| | | Crabgrass | Barnyard Grass** | Wild Oats | Rhizome Johnson-Grass | Yellow Foxtail | Cheat Grass | Blackgrass | Goosegrass |
| 1 | 0.28 | 98 | 100 | 99 | 95 | 100 | 90 | 75 | 85 |
| 1 | 0.11 | 90 | 100 | 60 | 90 | 96 | 45 | 75 | 80 |
| 1 | 0.04 | 70 | 85 | 0 | 65 | 80 | 0 | 15 | 45 |
| 1 | 0.02 | 0 | 0 | 0 | 20 | 25 | 0 | 0 | 0 |
| C-3 | 0.28 | 75 | 98 | 75 | 45 | 80 | 30 | 70 | 75 |
| C-3 | 0.11 | 25 | 80 | 20 | 30 | 35 | 0 | 0 | 50 |
| C-3 | 0.04 | 0 | 0 | 0 | 0 | 0 | 0 | 0 | 0 |
| C-3 | 0.02 | 0 | 0 | 0 | 0 | 0 | 0 | 0 | 0 |
| C-4 | 0.28 | 45 | 100 | 87 | 25 | 60 | 50 | 60 | 85 |
| C-4 | 0.11 | 0 | 92 | 30 | 0 | 60 | 0 | 20 | 40 |
| C-4 | 0.04 | 0 | 50 | 0 | 0 | 0 | 0 | 0 | 0 |
| C-4 | 0.02 | 0 | 0 | 0 | 0 | 0 | 0 | 0 | 0 |
| Check | 0 | 0 | 0 | 0 | 0 | 0 | 0 | 0 | 0 |

* q/cm² = microgram/square centimeter

** California Barnyardgrass.

[1] Reported as average of three replicates.

TABLE 5 (Cont'd)

| Compound No. | Dosage q/cm²* | Grasses % Phytotoxicity | | | | | |
|---|---|---|---|---|---|---|---|
| | | Sprangletop | Italian Rye Grass | Fall Panicum | Proso Millet | Field Corn** | Sorghum** |
| 1 | 0.28 | 92 | 95 | 96 | 99 | 99 | 100 |
| 1 | 0.11 | 82 | 87 | 82 | 90 | 96 | 92 |
| 1 | 0.04 | 15 | 30 | 15 | 35 | 90 | 70 |
| 1 | 0.02 | 0 | 0 | 0 | 0 | 20 | 0 |
| C-3 | 0.28 | 30 | 87 | 95 | 80 | 40 | 85 |
| C-3 | 0.11 | 0 | 30 | 35 | 40 | 20 | 30 |
| C-3 | 0.04 | 0 | 0 | 25 | 0 | 0 | 0 |
| C-3 | 0.02 | 0 | 0 | 0 | 0 | 0 | 0 |
| C-4 | 0.28 | 50 | 97 | 97 | 90 | 35 | 92 |
| C-4 | 0.11 | 0 | 70 | 72 | 35 | 0 | 30 |
| C-4 | 0.04 | 0 | 20 | 25 | 0 | 0 | 0 |
| C-4 | 0.02 | 0 | 0 | 0 | 0 | 0 | 0 |
| Check | 0 | 0 | 0 | 0 | 0 | 0 | 0 |

* q/cm² = micrograms/cm².
** Field corn and sorghum presents significant weed problems where crops are rotated.

TABLE 5A

CROPS

Post-Emergence Herbicidal Activity - Percent Control [1]

Low Dosage Tests

| Compound No. | Dosage q/cm²* | Grasses % Phytotoxicity | | | Broadleaf % Phytotoxicity | | |
|---|---|---|---|---|---|---|---|
| | | Barley | Rice | Anza Wheat | Sugar Beet | Cotton | Soybean |
| 1 | 0.28 | 98 | 100 | 98 | 0 | 5 | 40 |
| 1 | 0.11 | 50 | 99 | 80 | 0 | 0 | 10 |
| 1 | 0.04 | 0 | 30 | 20 | 0 | 0 | 0 |
| 1 | 0.02 | 0 | 0 | 0 | 0 | 0 | 0 |
| C-3 | 0.28 | 95 | 75 | 75 | 0 | 0 | 5 |
| C-3 | 0.11 | 30 | 20 | 0 | 0 | 0 | 1 |
| C-3 | 0.04 | 0 | 0 | 0 | 0 | 0 | 0 |
| C-3 | 0.02 | 0 | 0 | 0 | 0 | 0 | 0 |
| C-4 | 0.28 | 95 | 85 | 90 | 0 | 0 | 0 |
| C-4 | 0.11 | 50 | 20 | 50 | 0 | 0 | 0 |
| C-4 | 0.04 | 0 | 0 | 0 | 0 | 0 | 0 |
| C-4 | 0.02 | 0 | 0 | 0 | 0 | 0 | 0 |
| Check | 0 | 0 | 0 | 0 | 0 | 0 | 0 |

* $q/cm^2$ = microgram/square centimeter.
[1] Reported as average of four replicates.

From the results of the above test, it can be seen that Compound 1 was substantially more active than Compound C-3 against all fourteen of the weed grasses in the test and was substantially more active than Compound C-4 against twelve of the weed grasses and was at least as active as Compound C-4 with respect to the remaining two.

## Example 10

In this example, the compounds identified in Table D were respectively tested using the procedures described hereinbelow for pre-emergent and post-emergent activity against a variety of grasses and broad-leaf plants including one grain crop and one broad-leaf crop. The compounds tested are identified by compound number in Table D hereinbelow and were prepared using the general procedures described hereinabove.

## Pre-Emergent Herbicide Test

Pre-emergence herbicidal activity was determined in the following manner.

Test solutions of the respective compounds were prepared as follows:

355.5 mg of test compound was dissolved in 15 ml of acetone. 2 ml of acetone containing 110 mg of a non-ionic surfactant was added to the solution. The stock solution was then added to the appropriate amount of water, contained the same nonionic surfactant at a concentration of 625 mg/l, to give the desired test compound concentration in the spray solution.

Seeds of the test vegetation were planted in a pot of soil and the test solution was sprayed uniformly onto the soil surface at a dose of 4.4 micrograms/$cm^2$ unless otherwise specified in the following tables. The pot was watered and placed in a greenhouse. The pot was watered intermittently and observed for seedling emergence, health of emerging seedlings, etc., for a 3-week period. At the end of this period, the herbicidal effectiveness of the compound was rated based on the physiological observations. A 0-to-100 scale was used, 0 representing no phytotoxicity, 100 representing complete kill. The results of these tests are summarized in Table 6.

## Post-Emergent Herbicidal Test

The test compound was formulated in the same manner as described above for the pre-emergent test. This formulation was uniformly sprayed on 2 similar pots containing plants 2 to 3" (50 to 7.5 cm) tall (except wild oats, soybean and watergrass which were 3 to 4 inches tall) (approximately 15 to 25 plants per pot) at a dose of 4.4 microgram/$cm^2$ unless otherwise specified in the following tables. After the plants had dried, they were placed in a greenhouse and then watered intermittently at their bases as needed. The plants were observed periodically for phytotoxic effects and physiological and morphological responses to the treatment. After 3 weeks, the herbicidal effectiveness of the compound was rated based on these observations. A 0-to-100 scale was used, 0 representing no phytotoxicity, 100 representing complete kill. The results of these tests are summarized in Table 7.

TABLE D

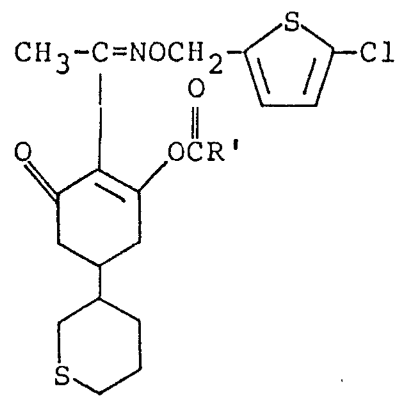

| Compound No. | R' | Appearance | Proton Chemical Shift w in ppm ($CDCl_3$) | *IR (neat) in $cm^{-1}$ |
|---|---|---|---|---|
| 1 | $CH_3$ | pale yellow oil | 1.00-2.60 (m, 14H), 1.93 (s, 3H), 2.00 (s, 3H), 5.07 (s, 2H), 6.73 (s, 2H) | 1775 |
| 2 | $CH_3CH_2$- | pale yellow oil | 1.10 (t, 3H), 1.00-2.60 (m, 16H), 1.93 (s, 3H), 5.10 (s, 2H), 6.77 (s, 2H) | 1775 |
| 3 | $CH_3(CH_2)_2$- | pale yellow oil | 0.93 (t, 3H), 1.00-2.70 (m, 18H), 1.92 (s, 3H), 5.07 (s, 2H), 6.73 (s, 2H) | 1770 |
| 4 | $CH_3(CH_2)_3$- | pale yellow oil | 0.92 (t, 3H), 1.00-2.60 (m, 20H), 1.93 (s, 3H), 5.10 (s, 2H), 6.78 (s, 2H) | 1775 |
| 5 | $CH_3(CH_2)_4$- | pale yellow oil | 0.89 (t, 3H), 1.00-2.70 (m, 22H), 1.92 (s, 3H), 5.09 (s, 2H), 6.78 (s, 2H) | 1770 |
| 6 | $(CH_3)_2CHCH_2$- | pale yellow oil | 0.80-2.60 (m, 20H), 1.92 (s, 3H), 5.08 (s, 2H), 6.77 (s, 2H) | 1775 |
| 7 | $CH_3(CH_2)_3(C_2H_5)CH$- | pale yellow oil | 0.90 (t, 6H), 1.00-2.70 (m, 23H), 1.91 (s, 3H), 5.07 (s, 2H), 6.75 (s, 2H) | 1770 |

*Infrared Spectra.

TABLE 6

Pre-Emergence Herbicidal Activity

Application Rate: 4.4 micrograms/$cm^2$, unless otherwise noted

| Compound No. | Broad-Leaf Plants % Phytotoxicity | | | | | Grasses % Phytotoxicity | | |
|---|---|---|---|---|---|---|---|---|
| | Lambsquarter | Mustard | Pigweed | Soybean | Barnyard Grass | Crabgrass | Wild Oats | Rice |
| 1 | 100 | 90 | 95 | 95 | 100 | 100 | 100 | 100 |
| 2 | 100 | 80 | 70 | 95 | 100 | 100 | 99 | 100 |
| 3 | 100 | 80 | 90 | 95 | 100 | 100 | 100 | 100 |
| 4 | - | 90 | 0 | 95 | 100 | 100 | - | 100 |
| 5 | 98 | 98 | 98 | 95 | 100 | 100 | 100 | 100 |
| 6 | 100 | 90 | 20 | 95 | 100 | 100 | 100 | 100 |
| 7 | 80 | 60 | 30 | 70 | 100 | 100 | 100 | 100 |

TABLE 7

Post-Emergence Herbicidal Activity

Application Rate: 4.4 micrograms/$cm^2$, unless otherwise noted

| Compound No. | Broad-Leaf Plants % Phytotoxicity | | | | | Grasses % Phytotoxicity | | |
|---|---|---|---|---|---|---|---|---|
| | Lambsquarter | Mustard | Pigweed | Soybean | Barnyard Grass | Crabgrass | Wild Oats | Rice |
| 1 | 30 | 60 | 50 | 90 | 100 | 100 | 100 | 100 |
| 2 | 20 | 70 | 30 | 90 | 100 | 100 | 100 | 100 |
| 3 | 20 | 70 | 80 | 90 | 100 | 100 | 100 | 100 |
| 4 | 40 | 70 | 30 | 95 | 100 | 100 | 100 | 100 |
| 5 | 20 | 80 | 70 | 90 | 100 | 100 | 100 | 100 |
| 6 | 30 | 80 | 60 | 90 | 100 | 100 | 98 | 100 |
| 7 | 60 | 70 | 60 | 95 | 100 | 100 | 100 | 100 |

## Claims

1. A compound having the formula:

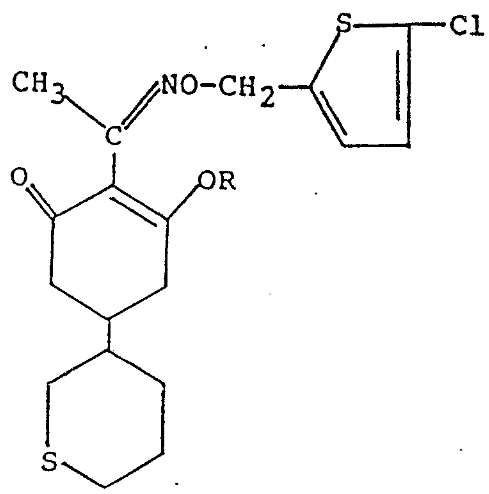

(I)

wherein R is hydrogen, a cation yielding a compatible salt, or $-CR^2$; wherein $R^2$ is alkyl having 1 through 10 carbon atoms; and tautomers and optical isomers of said compound.

2. The compound of Claim 1 which is 2-(1-((5-chlorothien-2-yl)methoxyimino)ethyl)-3-hydroxy-5-(tetrahydro-2H-thiopyran-3-yl)-cyclohex-2-en-1-one.

3. The compound of Claim 1 wherein $R^2$ is alkyl having 1 to 7 carbon atoms.

4. The compound of Claim 3 wherein said compound is 3-acetyloxy-2-(1-((5-chlorothien-2-yl)methoxyimino)ethyl)-5-(tetrahydro-2H-thiopyran-3-yl)-cyclohex-2-en-1-one.

5. A method for inhibiting the growth of axillary buds in tobacco plants which comprises applying to said tobacco plants or their growth medium an amount effective to inhibit the growth of axillary buds of one or more control agents selected from compounds claimed in any one of the preceding claims.

6. The method of Claim 5 wherein R is $-C(0)R^2$ wherein $R^2$ is alkyl having 1 through 3 carbon atoms.

7. The method of Claim 6 wherein $R^2$ is methyl, ethyl or n-propyl.

8. The method of any one of claims 5 to 7 wherein said growth regulating agent is applied at a rate of from 0.1-3.2 kg/ha.

9. The method of Claim 8 wherein said growth regulating agent is applied at a rate of from 0.4-2 kg/ha.

10. A tobacco sucker inhibiting composition comprising a compatible carrier and an amount effective to inhibit the growth of axillary buds in tobacco plants of one or more tobacco sucker control agents as claimed in any one of claims 1 to 4.

11. The composition of Claim 10 wherein said composition comprises from 0.01-0.32 wt % of said tobacco sucker control agent; from 0.001-0.0625 wt % of an emulsifier; from 0.08-3.3 wt % of an organic solvent and from 95-99 wt % water.

12. The composition of Claim 10 or 11 wherein $R^2$ is methyl.

13. A herbicidal composition comprising a herbicidally effective amount of one or more compounds according to any one of Claims 1 to 4 and an agriculturally acceptable carrier.

14. A herbicidal composition comprising 0.02-0.6 wt. % of one or more compounds according to any one of Claims 1 to 4; 0.001-0.15 wt. % of an emulsifier; 0.08-2.5 wt. % of an organic solvent and about 95 to 99 wt. % water.

15. A herbicidal composition as claimed in Claim 13, comprising a crop oil selected from soybean oils, paraffinic oils, olefinic oils and mixtures thereof.

16. The composition of Claim 15 wherein said composition comprises from 0.25-2 wt. % of said crop oil.

effective amount of one or more compounds according to any one of Claims 1 to 4 to the foliage or habitat of said plants.

18. A method as claimed, in Claim 17, wherein said plant in foxtail, Bermudagrass, volunteer sorghum, broad-leaf signalgrass, goosegrass, red rice, sprangletop, Johnsongrass or volunteer corn.

19. A method as claimed in Claim 17 or 18, wherein said compound is applied in an amount of 0.02 to 60kg/ha.

**Ammended claims in accordance with Rule 86(2) EPC.**

17. A method for controlling the growth of grassy plants which comprises applying a herbicidally effective amount of one or more compounds according to any one of Claims 1 to 4 to the foliage or habitat of said plants.

18. A method as claimed, in Claim 17, wherein said plant in foxtail, Bermudagrass, volunteer sorghum, broad-leaf signalgrass, goosegrass, red rice, sprangletop, Johnsongrass or volunteer corn.

19. A method as claimed in Claim 17 or 18, wherein said compound is applied in an amount of 0.02 to 60kg/ha.

European Patent Office

# EUROPEAN SEARCH REPORT

Application Number

EP 88 30 8855

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-4 624 696 (KEIL et al.)<br>* Claims *<br>--- | 1,5-19 | C 07 D 409/12<br>A 01 N 43/18 //<br>C 07 D 335/02 |
| D,Y | US-A-4 432 786 (W. LOH)<br>* Claims *<br>----- | 1,5-19 | |
| | | | TECHNICAL FIELDS SEARCHED (Int. Cl.4) |
| | | | C 07 D 409/00<br>C 07 D 335/00<br>C 07 D 333/00<br>A 01 N 43/00 |

The present search report has been drawn up for all claims

| Place of search | Date of completion of the search | Examiner |
|---|---|---|
| THE HAGUE | 15-12-1988 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document